(19) Europäisches Patentamt
European Patent Office
Office européen des brevets

(11) EP 4 239 051 A1

(12) **EUROPEAN PATENT APPLICATION**
published in accordance with Art. 153(4) EPC

(43) Date of publication:
**06.09.2023 Bulletin 2023/36**

(21) Application number: **21886267.0**

(22) Date of filing: **27.10.2021**

(51) International Patent Classification (IPC):
***C12M 1/00*** *(2006.01)* ***C12Q 1/02*** *(2006.01)*

(52) Cooperative Patent Classification (CPC):
**C12M 1/00; C12Q 1/02**

(86) International application number:
**PCT/JP2021/039652**

(87) International publication number:
**WO 2022/092150 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.11.2020 JP 2020183700**

(71) Applicant: **SEKISUI CHEMICAL CO., LTD.**
**Osaka-shi**
**Osaka**
**530-8565 (JP)**

(72) Inventors:
- **TOMITA, Yoshiki**
  **Mishima-gun, Osaka 618-0021 (JP)**
- **KOUNO, Takamasa**
  **Mishima-gun, Osaka 618-0021 (JP)**
- **IGUCHI, Hiroki**
  **Mishima-gun, Osaka 618-0021 (JP)**

(74) Representative: **Ter Meer Steinmeister & Partner Patentanwälte mbB**
**Nymphenburger Straße 4**
**80335 München (DE)**

Remarks:
The complete document including Reference Table(s) and the Sequence Listing(s) can be downloaded from the EPO website

# (54) MICROFLUIDIC CHIP, CELL ANALYSIS DEVICE, CELL ANALYSIS SYSTEM, AND CELL ANALYSIS METHOD

(57) Provided is a microfluidic chip capable of suppressing contamination while downsizing a device. A microfluidic chip 1 includes a chip main body 2, and an optical gas generation tape 8 attached to the chip main body 2 and configured to generate a gas by light irradiation, in which the chip main body 2 includes a microchannel 4, a liquid medium reservoir 5 provided in the middle of the microchannel 4, and a cell binding unit 6 provided on a downstream side of the liquid medium reservoir 5 in the microchannel 4, and a liquid medium stored in the liquid medium reservoir 5 is supplied to the cell binding unit 6 by the gas generated from the optical gas generation tape 8.

[FIG. 2.]

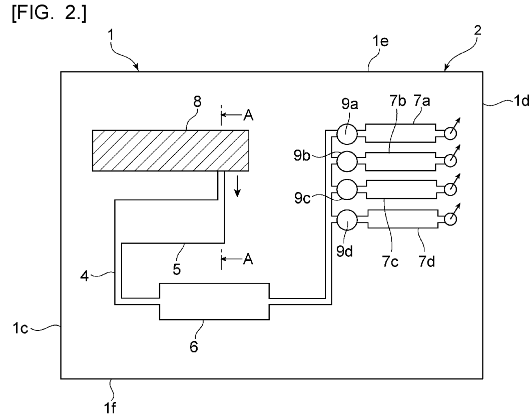


EP 4 239 051 A1

## Description

### TECHNICAL FIELD

The present invention relates to a microfluidic chip provided with a microchannel through which a fluid is fed, and a cell analysis device, a cell analysis system, and a cell analysis method that use the microfluidic chip.

### BACKGROUND ART

Conventionally, various tests and analyses have been attempted by controlling liquid delivery and reactions of various specimens or samples using a microfluidic chip provided with a microchannel through which a fluid is delivered.

Patent Document 1 below discloses a microfluidic device including at least two openings and a cell culture chamber. Patent Document 1 describes that the openings are connected to the cell culture chamber, and when liver cells and a scaffold are introduced into the cell culture chamber and cultured, liver cell maturation factors can be supplied from at least one opening to the cell culture chamber while forming a concentration gradient in the cell culture chamber.

### Related Art Document

### Patent Document

Patent Document 1: WO 2016/104541 A

### SUMMARY OF THE INVENTION

### PROBLEMS TO BE SOLVED BY THE INVENTION

However, in the microfluidic device of Patent Document 1, since it is necessary to supply a liquid medium or the like from the outside, there is a problem that size of the device is easily increased and contamination easily occurs.

An object of the present invention is to provide a microfluidic chip capable of suppressing contamination while downsizing the device, and a cell analysis device, a cell analysis system, and a cell analysis method that use the microfluidic chip.

### MEANS FOR SOLVING THE PROBLEMS

A microfluidic chip according to the present invention includes a chip main body, and an optical gas generation tape attached to the chip main body and configured to generate a gas by light irradiation, in which the chip main body includes a microchannel, a liquid medium reservoir provided in the middle of the microchannel, and a cell binding unit provided on a downstream side of the liquid medium reservoir in the microchannel, and a liquid medium stored in the liquid medium reservoir is supplied to the cell binding unit by the gas generated from the optical gas generation tape.

In a specific aspect of the microfluidic chip according to the present invention, the cell binding unit has a resin film, a resin constituting the resin film contains a poly(meth)acrylic acid ester skeleton or a polyvinyl acetal skeleton, a dispersion component $\gamma^d$ of surface free energy of the resin film is 24.5 mJ/$m^2$ or more and 45.0 mJ/$m^2$ or less, and a dipole component $\gamma^p$ of the surface free energy of the resin film is 1.0 mJ/$m^2$ or more and 20.0 mJ/$m^2$ or less.

In another specific aspect of the microfluidic chip according to the present invention, the cell binding unit has a resin film, and a resin constituting the resin film contains a poly(meth)acrylic acid ester skeleton or a polyvinyl acetal skeleton and a peptide moiety.

A cell analysis device according to the present invention includes the microfluidic chip configured according to the present invention, a light irradiation unit configured to irradiate the optical gas generation tape with light, and an analysis unit configured to analyze a cell culture solution that has passed through the cell binding unit.

A cell analysis system according to the present invention includes the microfluidic chip configured according to the present invention, a light irradiation unit, and an analysis unit, in which the light irradiation unit irradiates the optical gas generation tape with light, the liquid medium stored in the liquid medium reservoir is supplied to the cell binding unit by the gas generated from the optical gas generation tape by the light irradiation, and the analysis unit analyzes the cell culture solution that has passed through the cell binding unit.

A cell analysis method according to the present invention is a cell analysis method using the microfluidic chip configured according to the present invention, the method including the steps of: binding a cell to the cell binding unit; irradiating the optical gas generation tape with light; supplying the liquid medium stored in the liquid medium reservoir to the cell binding unit; and analyzing the cell culture solution that has passed through the cell binding unit.

## EFFECT OF THE INVENTION

According to the present invention, it is possible to provide a microfluidic chip capable of suppressing contamination while downsizing the device, and a cell analysis device, a cell analysis system, and a cell analysis method using the microfluidic chip.

## BRIEF DESCRIPTION OF DRAWINGS

[Fig. 1] Fig. 1 is a schematic perspective view illustrating an appearance of a microfluidic chip according to one embodiment of the present invention.
[Fig. 2] Fig. 2 is a schematic plan view for explaining a fluidic structure of the microfluidic chip according to one embodiment of the present invention.
[Fig. 3] Fig. 3 is an enlarged schematic sectional view taken along a line A-A in Fig. 2.
[Fig. 4] Fig. 4 is an enlarged schematic sectional view illustrating a portion provided with a cell binding unit in the microfluidic chip according to one embodiment of the present invention.
[Fig. 5] Fig. 5 is a schematic view illustrating a cell analysis device according to one embodiment of the present invention.

## MODE FOR CARRYING OUT THE INVENTION

Hereinafter, the present invention will be clarified by describing specific embodiments of the present invention with reference to the drawings.

[Microfluidic chip]

Fig. 1 is a schematic perspective view illustrating an appearance of a microfluidic chip according to one embodiment of the present invention. Fig. 2 is a schematic plan view for explaining a fluidic structure of the microfluidic chip according to one embodiment of the present invention. Fig. 3 is a schematic sectional view of a portion taken along the line A-A in Fig. 2.
As illustrated in Fig. 1, a microfluidic chip 1 has a rectangular plate-like shape. Specifically, the microfluidic chip 1 has a first main surface 1a and a second main surface 1b facing each other. Furthermore, the microfluidic chip 1 has first to fourth side surfaces 1c to 1f that connect the first main surface 1a and the second main surface 1b. The first side surface 1c and the second side surface 1d face each other. In addition, the third side surface 1e and the fourth side surface 1f also face each other. Note that the shape of the microfluidic chip 1 is not limited to such a rectangular plate-like shape, and may have other shapes.
The microfluidic chip 1 includes a chip main body 2. The chip main body 2 includes a substrate 3a and a cover member 3b provided on the substrate 3a. The substrate 3a is made of a synthetic resin injection molding. The cover member 3b is made of an elastomer or a synthetic resin. However, the substrate 3a and the cover member 3b may be made of other materials. Furthermore, the microfluidic chip 1 may be configured by laminating a plurality of synthetic resin sheets, and the structure and material thereof are not particularly limited.
A microchannel 4 through which a fluid is fed is provided inside the microfluidic chip 1. The microchannel 4 is a fine channel that produces a microeffect when a fluid is conveyed. In such a microchannel 4, the fluid is strongly affected by surface tension, and exhibits a behavior different from that of the fluid flowing in a normal large-sized fluidic path.
The sectional shape and size of the microchannel 4 are not particularly limited as long as the microchannel is a channel in which the microeffect described above occurs. For example, from a viewpoint of reducing fluidic resistance when a pump or gravity is used to flow a fluid in the microchannel 4, the sectional shape of the microchannel 4 is preferably 20 um or more, more preferably 50 um or more, still more preferably 100 um or more in terms of the dimension of the smaller side when the sectional shape is generally rectangular (including square). From a viewpoint of further downsizing the microfluidic chip 1, the dimension of the smaller side is preferably 5 mm or less, more preferably 1 mm or less, still more preferably 500 um or less.
Furthermore, when the sectional shape of the microchannel 4 is generally circular, the diameter (the minor axis in a case of ellipse) is preferably 20 um or more, more preferably 50 um or more, still more preferably 100 um or more. From the viewpoint of further downsizing the microfluidic chip 1, the diameter (the minor axis in case of ellipse) is preferably 5 mm or less, more preferably 1 mm or less, still more preferably 500 um or less.
On the other hand, for example, in a case where a capillary phenomenon is effectively utilized for fluid flow

through the microchannel 4, when the sectional shape of the microchannel 4 is generally rectangular (including square), the dimension of the smaller side is preferably 5 um or more, more preferably 10 um or more, and still more preferably 20 um or more. In addition, the size of the smaller side is preferably 200 um or less, and more preferably 100 um or less.

In the microfluidic chip 1, the fluidic structure illustrated in Fig. 2 is configured. As shown in Fig. 2, the microchannel 4 is provided with a liquid medium reservoir 5, a cell binding unit 6, and recovery units 7a to 7d.

The liquid medium reservoir 5 is provided in the middle of the microchannel 4. The liquid medium reservoir 5 is a reservoir for storing a liquid medium to be supplied to the cell binding unit 6. The liquid medium is not particularly limited, but for example, a serum-free medium can be used.

The volume of the liquid medium reservoir 5 is not particularly limited, but ranges, for example, from 0.1 mL or more to 30 mL or less.

The cell binding unit 6 is provided on the downstream side of the liquid medium reservoir 5 in the microchannel 4. In the cell binding unit 6, cells introduced from a cell introduction unit (not illustrated) can be bound. In the microfluidic chip 1, the cells can be cultured by supplying the liquid medium stored in the liquid medium reservoir 5 to the cell binding unit 6 to which the cells are bound.

The volume of the cell binding unit 6 is not particularly limited, but is, for example, 0.05 mL or more and 10 mL or less. Furthermore, a bottom area of the cell binding unit 6 is not particularly limited, but is, for example, 0.5 $cm^2$ or more and 20 $cm^2$ or less.

The recovery units 7a to 7d are provided on the downstream side of the cell binding unit 6 in the microchannel 4. A cell culture solution having passed through the cell binding unit 6 can be recovered by the recovery units 7a to 7d and subjected to analysis. Furthermore, in the present embodiment, the four recovery units 7a to 7d are provided, but only one recovery unit may be provided, and there is no particular limitation.

However, when the plurality of recovery units 7a to 7d are provided as in the present embodiment, for example, the cell culture solution after one day can be recovered in the recovery unit 7a, the cell culture solution after two days can be recovered in the recovery unit 7b, the cell culture solution after three days can be recovered in the recovery unit 7c, and the cell culture solution after four days can be recovered in the recovery unit 7d, and they can be analyzed respectively. At this time, for example, the respective cell culture solutions can be recovered using valves 9a to 9d so as not to be mixed.

Meanwhile, as illustrated in Fig. 3, in the present embodiment, an optical gas generation tape 8 is attached to the chip main body 2. More specifically, the optical gas generation tape 8 is attached so as to seal an opening at the upstream end of the microchannel 4. The optical gas generation tape 8 is disposed upstream of the liquid medium reservoir 5.

The optical gas generation tape 8 is a tape that generates a gas by being irradiated with light. Using the gas generated from the optical gas generation tape 8, the liquid medium stored in the liquid medium reservoir 5 is supplied to the cell binding unit 6. In addition, the cell culture solution that has passed through the cell binding unit 6 can be fed to the recovery units 7a to 7d and recovered by the gas generated from the optical gas generation tape 8.

A thickness of the optical gas generation tape 8 is not particularly limited, but is preferably 5 um or more, more preferably 10 um or more, and preferably 5 mm or less, more preferably 500 um or less.

The optical gas generation tape 8 preferably contains a photoresponsive gas generating agent.

The photoresponsive gas generating agent generates a gas when irradiated with light. Examples of the photoresponsive gas generating agent include azo compounds and azide compounds.

Examples of the azo compounds include 2,2'-azobis(N-cyclohexyl-2 methylpropionamide), 2,2'-azobis[N-(2-methylpropyl)-2 methylpropionamide], 2,2'-azobis(N-butyl-2 methylpropionamide), 2,2'-azobis[N-(2-methylethyl)-2 methylpropionamide], 2,2'-azobis(N-hexyl-2 methylpropionamide), 2,2'-azobis(N-propyl-2 methylpropionamide), 2,2'-azobis(N-ethyl-2 methylpropionamide), 2,2'-azobis{2-methyl-N-[1,1-bis(hydroxymethyl)-2 hydroxyethyl]propionamide}, 2,2'-azobis{2-methyl-N-[2-(1-hydroxybutyl)]propionamide}, 2,2'-azobis[2-methyl-N-(2-hydroxyethyl)propionamide], 2,2'-azobis[N-(2-propenyl)-2 methylpropionamide], 2,2'-azobis[2-(5-methyl-2 imidazoilin-2-yl)propane]dihydrochloride, 2,2'-azobis[2-(2-imidazoilin-2-yl)propane]dihydrochloride, 2,2'-azobis[2-(2-imidazoilin-2-yl)propane]disulphate dihydrochloride, 2,2'-azobis[2-(3,4,5,6-tetrahydropyrimidin-2-yl)propane]dihydrochloride, 2,2'-azobis{2-[1-(2-hydroxyethyl)-2-imidazoylin-2-yl]propane}dihydrochloride, 2,2'-azobis[2-(2-imidazoylin-2-yl)propane], 2,2'-azobis(2-methylpropionamidine)hydrochloride, 2,2'-azobis(2-aminopropane)dihydrochloride, 2,2'-azobis[N-(2-carboxyacyl)-2-methyl-propionamidine], 2,2'-azobis{2-[N-(2-carboxyethyl)amidine]propane}, 2,2'-azobis(2-methylpropionamidoxime), dimethyl 2,2'-azobis(2-methylpropionate), dimethyl 2,2'-azobisisobutyrate, 4,4'-azobis(4-cyanocarbonic acid), and 4,4'-azobis(4-cyanopentanoic acid).

Examples of the azide compounds include 3-azidomethyl-3-methyloxetane, terephthalazide, and a polymer having an azide group. A specific example of the polymer having an azide group is glycidyl azide polymer. The glycidyl azide polymer is obtained, for example, by ring-opening polymerization of p-tert-butylbenzazide and 3-azidomethyl-3-methyloxetane.

Furthermore, an adhesive unit of the optical gas generation tape 8 can be made of, for example, a (meth)acrylic

adhesive. A thickness of the adhesive unit of the optical gas generation tape 8 is preferably 1 um or more, more preferably 5 um or more, still more preferably 10 um or more, and preferably 200 um or less, more preferably 100 um or less, still more preferably 50 um or less.

In the present embodiment, the liquid medium reservoir 5 is provided inside the microfluidic chip 1. Furthermore, the liquid medium stored in the liquid medium reservoir 5 can be supplied to the cell binding unit 6 by the gas generated from the optical gas generation tape 8. Accordingly, in the case of the microfluidic chip 1 of the present embodiment, it is not necessary to provide a pump for generating gas or a liquid medium supply unit outside, so that the external device can be simplified, and the device is unlikely to be upsized. In addition, since the liquid medium can be stored inside and the liquid medium can be fed in a state where the upper end of the microchannel 4 is sealed by the optical gas generation tape 8, contamination from the outside can also be suppressed. Therefore, according to the microfluidic chip 1, it is possible to suppress contamination while downsizing the device.

Details of the cell binding unit 6 will be described below.

(Cell binding unit)

Fig. 4 is an enlarged schematic sectional view illustrating a portion provided with a cell binding unit in the microfluidic chip according to one embodiment of the present invention.

As shown in Fig. 4, the cell binding unit 6 has a resin film 10. The resin film 10 is used for culturing cells. The resin film 10 is used as a scaffold for cells when the cells are cultured.

Examples of the cells include animal cells such as a human, a mouse, a rat, a pig, a cow, and a monkey. In addition, examples of the cells include somatic cells, and include stem cells, progenitor cells, and mature cells. The somatic cells may be cancer cells.

Examples of the mature cells include nerve cells, cardiomyocytes, retinal cells, and hepatocytes.

Examples of the stem cells include mesenchymal stem cells (MSC), iPS cells, ES cells, Muse cells, embryonic cancer cells, embryonic germ stem cells, and mGS cells.

The resin film 10 is formed of a scaffold material for cell culture containing a synthetic resin. The resin film 10 may be formed of a scaffold material for cell culture using a natural polymer material. Although, the scaffold material for cell culture using a natural polymer material can enhance fixability of cells after seeding, it is expensive, has large variations between lots due to a naturally derived substance, or has safety concerns due to animal-derived components. Furthermore, storage at room temperature is difficult, and freezing storage is often required. Therefore, the resin film 10 is preferably formed of a scaffold material for cell culture containing a synthetic resin. In this case, as compared with a scaffold material using a natural polymer material, the scaffold material for cell culture containing a synthetic resin has good operability, is inexpensive, has little variation between lots, and is excellent in safety. In addition, since storage at room temperature is possible, handleability can be further enhanced when the microfluidic chip 1 is used.

In the present invention, it is preferable that the scaffold material for cell culture constituting the resin film 10 does not substantially contain an animal-derived raw material. By substantially not containing any animal-derived raw material, it is possible to provide the resin film 10 having high safety and little variation in quality during manufacturing. The phrase "substantially not containing any animal-derived raw material" means that the animal-derived raw material in the scaffold material for cell culture is 3 wt% or less. In the scaffold material for cell culture, the animal-derived raw material in the scaffold material for cell culture is preferably 1 wt% or less, and most preferably 0 wt%. In other words, it is most preferable that the scaffold material for cell culture constituting the resin film 10 does not contain any animal-derived raw material.

In the present invention, a dispersion component $\gamma^d$ of surface free energy of the resin film 10 is preferably 24.5 mJ/m$^2$ or more, more preferably 28.0 mJ/m$^2$ or more, and preferably 45.0 mJ/m$^2$ or less, more preferably 38.0 mJ/m$^2$ or less.

Furthermore, a dipole component $\gamma^p$ of the surface free energy of the resin film 10 is preferably 1.0 mJ/m$^2$ or more, more preferably 2.5 mJ/m$^2$ or more, and preferably 20.0 mJ/m$^2$ or less, more preferably 10.0 mJ/m$^2$ or less.

When the surface free energy of the resin film 10 is within the above ranges, the resin film has moderate hydrophilicity and strength, and therefore the fixability after seeding of cells can be further enhanced. In particular, in a serum-free medium culture not containing feeder cells or adhesion proteins, an initial fixing rate after cell seeding is further improved.

The dispersion component $\gamma^d$ and the dipole component $\gamma^p$ of the surface free energy are calculated using the theoretical formula of Kaelble-Uy. The theoretical formula of Kaelble-Uy is based on the assumption that the total surface free energy $\gamma$ is the sum of the dispersion component $\gamma^d$ and the dipole component $\gamma^p$ as expressed by Formula (1) below.

[Mathematical 1]

$$\gamma = \gamma^{d} + \gamma^{p} \qquad (1)$$

In addition, in the theoretical formula of Kaelble-Uy, when the surface free energy of a liquid is $\gamma_l$(mJ/m$^2$), the surface free energy of a solid is $\gamma_s$(mJ/m$^2$), and the contact angle is $\theta$ (°), Formula (2) below is established.
[Mathematical 2]

$$\gamma_l (1 + \cos\theta) = 2\sqrt{\gamma_s^d \gamma_l^d} + 2\sqrt{\gamma_s^p \gamma_l^p} \qquad (2)$$

Accordingly, the dispersion component $\gamma^d$ and the dipole component $\gamma^p$ of the surface free energy of the resin film 10 can be obtained by measuring the contact angle $\theta$ with respect to the resin film 10 using two types of liquids whose surface free energy $\gamma_l$ is known and by solving simultaneous equations of $\gamma_s{}^d$ and $\gamma_s{}^p$.

Furthermore, in the present invention, pure water and diiodomethane are used as the two types of liquids whose surface free energy $\gamma_l$ is known.

The contact angle $\theta$ is measured as follows using a contact angle meter (for example, "DMo-701" manufactured by Kyowa Interface Science Co., Ltd.).

Pure water or diiodomethane (1 uL) is dropped onto the surface of the resin film 10. An angle formed between the pure water and the resin film 10 (30 seconds after dropping) is defined as the contact angle $\theta$ with respect to the pure water. Similarly, an angle formed between diiodomethane and the resin film 10 (30 seconds after dropping) is defined as the contact angle $\theta$ with respect to the diiodomethane.

From a viewpoint of suitably adjusting the dispersion component $\gamma^d$ and the dipole component $\gamma^p$ of the surface free energy, the synthetic resin constituting the resin film 10 preferably has at least one of a polyvinyl acetal skeleton and a poly(meth)acrylic acid ester skeleton.

In this case, the synthetic resin may be a resin having a polyvinyl acetal skeleton, a resin having a poly(meth)acrylic acid ester skeleton, or a resin having both a polyvinyl acetal skeleton and a poly(meth)acrylic acid ester skeleton.

Among them, from a viewpoint of further enhancing the adhesiveness to cells, the synthetic resin is preferably a resin having a polyvinyl acetal skeleton or a resin having a polyvinyl acetal skeleton and a poly(meth)acrylic acid ester skeleton. The synthetic resin is preferably a resin having at least a polyvinyl acetal skeleton.

Synthetic resin having a polyvinyl acetal skeleton;

The scaffold material for cell culture constituting the resin film 10 preferably contains a synthetic resin having a polyvinyl acetal skeleton.

In the present specification, a "synthetic resin having a polyvinyl acetal skeleton" may be described as a "polyvinyl acetal resin X".

The polyvinyl acetal resin X has an acetal group, an acetyl group, and a hydroxyl group in a side chain.

When the polyvinyl acetal resin X is synthesized, at least a step of acetalizing polyvinyl alcohol with an aldehyde is included.

The aldehyde used for acetalization of polyvinyl alcohol for obtaining the polyvinyl acetal resin X is not particularly limited. Examples of the aldehyde include an aldehyde having 1 to 10 carbon atoms. The aldehyde may have a chain aliphatic group, a cyclic aliphatic group, or an aromatic group. The aldehyde may be a chain aldehyde or a cyclic aldehyde.

Examples of the aldehyde include formaldehyde, acetaldehyde, propionaldehyde, butyraldehyde, pentanal, hexanal, heptanal, octanal, nonanal, decanal, acrolein, benzaldehyde, cinnamaldehyde, perillaldehyde, formylpyridine, formylimidazole, formylpyrrole, formylpiperidine, formyltriazole, formyltetrazole, formylindole, formylisoindole, formylpurine, formylbenzimidazole, formylbenzotriazole, formylquinoline, formylisoquinoline, formylquinoxaline, formylcinnoline, formylpteridine, formylfuran, formyloxolane, formyloxane, formylthiophene, formylthiolane, formylthiane, formyladenine, formylguanine, formylcytosine, formylthymine, and formyluracil. Only one kind of the aldehyde may be used, or two or more kinds thereof may be used in combination.

The aldehyde is preferably formaldehyde, acetaldehyde, propionaldehyde, butyraldehyde, or pentanal, and more preferably butyraldehyde. Accordingly, the polyvinyl acetal skeleton is preferably a polyvinyl butyral skeleton. The polyvinyl acetal resin X is preferably a polyvinyl butyral resin.

A vinyl compound may be copolymerized with the polyvinyl acetal resin X. In other words, the polyvinyl acetal resin X may be a copolymer of a structural moiety of a polyvinyl acetal resin and the vinyl compound. In the present invention, the polyvinyl acetal resin copolymerized with the vinyl compound is also referred to as a polyvinyl acetal resin.

The vinyl compound is a compound having a vinyl group ($H_2C{=}CH$-). The vinyl compound may be a polymer

with a structural moiety having a vinyl group.

The copolymer may be a block copolymer of a polyvinyl acetal resin and a vinyl compound, or may be a graft copolymer in which a vinyl compound is grafted to a polyvinyl acetal resin. The copolymer is preferably a graft copolymer.

Examples of the vinyl compound include ethylene, allylamine, vinylpyrrolidone, vinylimidazole, maleic anhydride, maleimide, itaconic acid, (meth)acrylic acid, vinylamine, and (meth)acrylic acid ester. These vinyl compounds may be used singly or in combination of two or more kinds thereof.

From a viewpoint of further enhancing cell adhesiveness, the polyvinyl acetal resin X preferably has a Bronsted basic group or a Bronsted acidic group, and more preferably has a Bronsted basic group. In other words, it is preferable that a part of the polyvinyl acetal resin X is modified with a Bronsted basic group or a Bronsted acidic group, and it is more preferable that a part of the polyvinyl acetal resin X is modified with a Bronsted basic group.

An acetalization degree of the polyvinyl acetal resin X (a butyralization degree in the case of the polyvinyl butyral resin) is preferably 50 mol% or more, more preferably 60 mol% or more, and preferably 90 mol% or less, more preferably 80 mol% or less. When the acetalization degree is the above lower limit or more, the fixability of cells is further excellent, and cell proliferation can be performed more efficiently. When the acetalization degree is the above upper limit or less, solubility in a solvent can be further improved.

Synthetic resin having a poly(meth)acrylic acid ester skeleton;

The scaffold material for cell culture constituting the resin film 10 may contain a synthetic resin having a poly(meth)acrylic acid ester skeleton.

In the present specification, a "synthetic resin having a poly(meth)acrylic acid ester skeleton" may be referred to as a "poly(meth)acrylic acid ester resin X". Accordingly, the poly(meth)acrylic acid ester resin X is a resin having a poly(meth)acrylic acid ester skeleton.

The poly(meth)acrylic acid ester resin X is obtained by polymerization of a (meth)acrylic acid ester or copolymerization of a (meth)acrylic acid ester with another monomer.

Examples of the (meth)acrylic acid ester include (meth)acrylic acid alkyl ester, (meth)acrylic acid cyclic alkyl ester, (meth)acrylic acid aryl ester, (meth)acrylamides, (meth)acrylic acid polyethylene glycols, and (meth)acrylic acid phosphorylcholine.

Examples of the (meth)acrylic acid alkyl ester include methyl(meth)acrylate, ethyl(meth)acrylate, n-propyl(meth)acrylate, isopropyl(meth)acrylate, n-butyl(meth)acrylate, isobutyl(meth)acrylate, t-butyl(meth)acrylate, n-octyl(meth)acrylate, isooctyl(meth)acrylate, 2-ethylhexyl(meth)acrylate, nonyl(meth)acrylate, isononyl(meth)acrylate, decyl(meth)acrylate, isodecyl(meth)acrylate, lauryl(meth)acrylate, stearyl(meth)acrylate, and isotetradecyl(meth)acrylate.

Furthermore, the (meth)acrylic acid alkyl ester may be substituted with a substituent such as an alkoxy group having 1 to 3 carbon atoms and a tetrahydrofurfuryl group. Examples of such (meth)acrylic acid alkyl ester include methoxyethyl acrylate and tetrahydrofurfuryl acrylate.

Examples of the (meth)acrylic acid cyclic alkyl ester include cyclohexyl(meth)acrylate and isobornyl(meth)acrylate.

Examples of the (meth)acrylic acid aryl ester include phenyl(meth)acrylate and benzyl(meth)acrylate.

Examples of the (meth)acrylamides include (meth)acrylamide, N-isopropyl(meth)acrylamide, N-tert-butyl(meth)acrylamide, N,N'-dimethyl(meth)acrylamide, (3-(meth)acrylamidopropyl)trimethylammonium chloride, 4-(meth)acryloylmorpholine, 3-(meth)acryloyl-2-oxazolidinone, N-[3-(dimethylamino)propyl](meth)acrylamide, N-(2-hydroxyethyl)(meth)acrylamide, N-methylol(meth)acrylamide, and 6-(meth)acrylamidohexanoic acid.

Examples of the (meth)acrylic acid polyethylene glycols include methoxy-polyethylene glycol(meth)acrylate, ethoxy-polyethylene glycol(meth)acrylate, hydroxy-polyethylene glycol(meth)acrylate, methoxy-diethylene glycol(meth)acrylate, ethoxy-diethylene glycol(meth)acrylate, hydroxy-diethylene glycol(meth)acrylate, methoxy-triethylene glycol(meth)acrylate, ethoxy-triethylene glycol(meth)acrylate, and hydroxy-triethylene glycol(meth)acrylate.

An example of the (meth) acrylic acid phosphorylcholine is 2-(meth)acryloyloxyethyl phosphorylcholine.

As another monomer copolymerized with the (meth)acrylic acid ester, a vinyl compound is suitably used. Examples of the vinyl compound include ethylene, allylamine, vinylpyrrolidone, maleic anhydride, maleimide, itaconic acid, (meth)acrylic acid, vinylamine, and (meth)acrylic acid ester. Only one kind of the vinyl compound may be used, or two or more kinds thereof may be used in combination.

In the present specification, "(meth)acrylic" means "acrylic" or "methacrylic", and " (meth)acrylate" means "acrylate" or "methacrylate".

Similarly to a synthetic resin X having a polyvinyl acetal skeleton, a synthetic resin X having a poly(meth)acrylic acid ester skeleton preferably partially has a Bronsted basic group or a Bronsted acidic group. In that case, the adhesiveness and proliferation of cells can be further enhanced. The Bronsted basic group may be contained in a part of the synthetic resin X having a poly(meth)acrylic acid ester skeleton, and in that case, a monomer having a Bronsted basic group may be copolymerized or grafted.

In the synthetic resin X having a poly(meth)acrylic acid ester skeleton, the total of the structure derived from a (meth)acrylic acid alkyl ester, the structure derived from a (meth)acrylic acid cyclic alkyl ester, and the structure derived

from a (meth)acrylic acid aryl ester contained in the structural unit is preferably 50 wt% or more, more preferably 60 wt% or more, and still more preferably 70 wt% or more. By setting the ratio of these hydrophobic (meth)acrylic acid ester-derived structures to the above lower limit value or more, the cell adhesiveness can be further enhanced.

The upper limit value of the total of the structure derived from a (meth)alkyl acid alkyl ester, the structure derived from a (meth)acrylic acid cyclic alkyl ester, and the structure derived from a (meth)acrylic acid aryl ester contained in the structural unit of the synthetic resin X having a poly(meth)acrylic acid ester skeleton is not particularly limited, and may be 100 wt% or less.

Glass transition temperature of the synthetic resin X having a poly(meth)acrylic acid ester skeleton is preferably 40°C or more, more preferably 45°C or more, and still more preferably 50°C or more. By setting the glass transition temperature to the above lower limit value or more, the cell adhesiveness can be further enhanced. The upper limit value of the glass transition temperature is not particularly limited, but may be, for example, 300°C or less.

Synthetic resin having a peptide moiety;

The scaffold material for cell culture constituting the resin film 10 preferably contains a synthetic resin having a polyvinyl acetal skeleton or a poly(meth)acrylic acid ester skeleton and a peptide moiety (hereinafter, a synthetic resin X having a peptide moiety). The synthetic resin X having a peptide moiety can be obtained by reacting a synthetic resin, a linker, and a peptide. The synthetic resin X having a peptide moiety is preferably a peptide-containing polyvinyl acetal resin having a polyvinyl acetal resin moiety, a linker moiety, and a peptide moiety, and more preferably a peptide-containing polyvinyl butyral resin having a polyvinyl butyral resin moiety, a linker moiety, and a peptide moiety. Only one kind of the synthetic resin X having a peptide moiety may be used, or two or more kinds thereof may be used in combination.

The peptide moiety is preferably composed of 3 or more amino acids, more preferably 4 or more amino acids, still more preferably 5 or more amino acids, and preferably 10 or less amino acids, more preferably 6 or less amino acids. When the number of amino acids constituting the peptide moiety is the above lower limit or more and the above upper limit or less, the adhesiveness to cells after seeding can be further enhanced and proliferation rate of cells can be further enhanced.

The peptide moiety preferably has a cell adhesive amino acid sequence. Furthermore, the cell adhesive amino acid sequence refers to an amino acid sequence whose cell adhesion activity has been confirmed by a phage display method, a Sepharose bead method, or a plate coating method. As the phage display method, for example, the method described in "The Journal of Cell Biology, Volume 130, Number 5, September 1995 1189-1196" can be used. As the Sepharose bead method, for example, the method described in "Protein, Nucleic Acid and Enzyme, Vol. 45, No. 15 (2000) 2477" can be used. As the plate coating method, for example, the method described in "Protein, Nucleic Acid and Enzyme, Vol. 45, No. 15 (2000) 2477" can be used.

Examples of the cell adhesive amino acid sequence include RGD sequence (Arg-Gly-Asp), YIGSR sequence (Tyr-Ile-Gly-Ser-Arg), PDSGR sequence (Pro-Asp-Ser-Gly-Arg), HAV sequence (His-Ala-Val), ADT sequence (Ala-Asp-Thr), QAV sequence (Gln-Ala-Val), LDV sequence (Leu-Asp-Val), IDS sequence (Ile-Asp-Ser), REDV sequence (Arg-Glu-Asp-Val), IDAPS sequence (Ile-Asp-Ala-Pro-Ser), KQAGDV sequence (Lys-Gln-Ala-Gly-Asp-Val), and TDE sequence (Thr-Asp-Glu). Furthermore, examples of the cell adhesive amino acid sequence include the sequences described in "Pathophysiology, Vol. 9, No. 7, p. 527 to 535, 1990" and "Osaka Women's and Children's Hospital Journal, Vol. 8, No. 1, p. 58 to 66, 1992". The peptide moiety may have only one kind of the cell-adhesive amino acid sequence or two or more kinds thereof.

The cell adhesive amino acid sequence preferably has at least one of the cell adhesive amino acid sequences, more preferably has at least an RGD sequence, a YIGSR sequence, or a PDSGR sequence, and still more preferably has at least an RGD sequence represented by Formula (3) below. In this case, the adhesiveness to cells after seeding can be further enhanced and the proliferation rate of the cells can be further enhanced.

Arg-Gly-Asp-X ··· Formula (3)

In the above Formula (3), X represents Gly, Ala, Val, Ser, Thr, Phe, Met, Pro, or Asn.

The peptide moiety may be linear or may have a cyclic peptide skeleton. The cyclic peptide skeleton is a cyclic skeleton composed of a plurality of amino acids. From a viewpoint of more effectively exhibiting the effects of the present invention, the cyclic peptide skeleton is preferably composed of 4 or more amino acids, preferably 5 or more amino acids, and preferably 10 or less amino acids.

In the synthetic resin X having a peptide moiety, content of the peptide moiety is preferably 0.1 mol% or more, more preferably 1 mol% or more, still more preferably 5 mol% or more, and particularly preferably 10 mol% or more. In the synthetic resin X having a peptide moiety, the content of the peptide moiety is preferably 60 mol% or less, more preferably 50 mol% or less, still more preferably 35 mol% or less, and particularly preferably 25 mol% or less. When the content of the peptide moiety is the above lower limit or more, the adhesiveness to cells after seeding can be further

enhanced and the proliferation rate of cells can be further enhanced. When the content of the peptide moiety is the above upper limit or less, production costs can be suppressed. Note that the content (mol%) of the peptide moiety is substance amount of the peptide moiety with respect to the sum of the substance amounts of the structural moieties constituting the synthetic resin X having a peptide moiety.

The content of the peptide moiety can be measured by FT-IR or LC-MS.

In the synthetic resin X having a peptide moiety, the synthetic resin moiety and the peptide moiety are preferably bonded via a linker. In other words, the synthetic resin X having a peptide moiety is preferably a synthetic resin having a peptide moiety and a linker moiety. Only one kind of the linker may be used, or two or more kinds thereof may be used in combination.

The linker is preferably a compound having a functional group capable of condensing with a carboxyl group or an amino group of the peptide. Examples of the functional group capable of condensing with a carboxyl group or an amino group of the peptide include a carboxyl group, a thiol group, and an amino group. From a viewpoint of favorably reacting with the peptide, the linker is preferably a compound having a carboxyl group. As the linker, the vinyl compound described above can also be used.

Examples of the linker having a carboxyl group include (meth)acrylic acid and carboxyl group-containing acrylamide. When a carboxylic acid (a carboxylic acid monomer) having a polymerizable unsaturated group is used as the linker having a carboxyl group, the carboxylic acid monomer can be polymerized by graft polymerization when reacting the linker with the synthetic resin X having a peptide moiety, so that the number of carboxyl groups that can be reacted with the peptide can be increased.

The scaffold material for cell culture constituting the resin film 10 may contain components other than a synthetic resin having a polyvinyl acetal skeleton and a poly(meth)acrylic acid ester skeleton. Examples of other components include polysaccharides, cellulose, synthetic peptides, polyolefin resins, polyether resins, polyvinyl alcohol resins, polyesters, epoxy resins, polyamide resins, polyimide resins, polyurethane resins, polycarbonate resins, polysaccharides, cellulose, polypeptides, and synthetic peptides.

From a viewpoint of effectively exhibiting the effects of the present invention, the content of other components is preferably as small as possible. In 100 wt% of the scaffold material for cell culture, the content of the component is preferably 10 wt% or less, more preferably 5 wt% or less, still more preferably 2.5 wt% or less, particularly preferably 1 wt% or less, and most preferably 0 wt% (free).

However, the scaffold material for cell culture constituting the resin film 10 may be composed of a vinyl polymer other than the synthetic resin described above. The vinyl polymer is a polymer of a compound having a vinyl group or a vinylidene group. Examples of the vinyl polymer include polyvinyl alcohol derivatives, poly(meth)acrylic acid esters, polyvinylpyrrolidone, polystyrene, and ethylene-vinyl acetate copolymers.

The area of the resin film 10 in plan view can be, for example, 0.1 $cm^2$ or more and 10 $cm^2$ or less. The thickness of the resin film 10 can be, for example, 5 nm or more and 500 nm or less.

[Cell analysis device, cell analysis system, and cell analysis method]

Fig. 5 is a schematic view showing a cell analysis device according to one embodiment of the present invention. As illustrated in Fig. 5, a cell analysis device 21 includes the microfluidic chip 1, a light irradiation unit 22, and an analysis unit 23. The light irradiation unit 22 is provided to irradiate the optical gas generation tape 8 with light. Furthermore, the analysis unit 23 is provided to analyze the cell culture solution that has passed through the cell binding unit 6 in the microfluidic chip 1.

In the cell analysis method of the present invention, an analysis device such as the cell analysis device 21 can be used. In the analysis method using the cell analysis device 21, first, cells are bound to the cell binding unit 6. Next, the optical gas generation tape 8 is irradiated with light, and the liquid medium stored in the liquid medium reservoir 5 is supplied to the cell binding unit 6 by the gas generated from the optical gas generation tape 8 by the light irradiation. Then, the cell culture solution that has passed through the cell binding unit 6 is recovered by the recovery units 7a to 7d and analyzed by the analysis unit 23.

In addition, a cell analysis system according to the present invention also includes the microfluidic chip 1, the light irradiation unit 22, and the analysis unit 23. Also in this system, the optical gas generation tape 8 is irradiated with light by the light irradiation unit 22, and the liquid medium stored in the liquid medium reservoir 5 is supplied to the cell binding unit 6 by the gas generated from the optical gas generation tape 8 by the light irradiation. Then, the cell culture solution that has passed through the cell binding unit 6 is recovered by the recovery units 7a to 7d and analyzed by the analysis unit 23.

In the cell analysis device, the cell analysis system, and the cell analysis method according to the present invention, since the microfluidic chip 1 is used, it is possible to suppress contamination while downsizing the device, and accuracy of analysis can also be improved.

(Experimental examples)

In Experimental Examples 1 to 5 and Reference Experimental Example, the microfluidic chip 1 having a fluidic structure shown in Fig. 2 was produced. The microfluidic chip 1 was prepared by bonding the cover member 3b to the substrate 3a that is an injection molding body made of a cycloolefin polymer. The microfluidic chip 1 was provided with the microchannel 4, the liquid medium reservoir 5, the cell binding unit 6, the recovery units 7a to 7d, and the optical gas generation tape 8. The microchannel 4 had a width of 0.5 mm, a depth of 1 mm, and a length of 20 mm. In the liquid medium reservoir 5, 4 ml of a liquid medium was sealed. The bottom area of the cell binding unit 6 was 3.8 $cm^2$. Resins constituting the resin film 10 on the surface of the cell binding unit 6 in the microfluidic chip 1 were prepared as follows.

Experimental Example 1;

Into a reactor equipped with a stirrer, 2700 mL of ion-exchanged water, and 300 parts by weight of polyvinyl alcohol having an average polymerization degree of 250 and a saponification degree of 99 mol% were charged, and heated and dissolved with stirring to obtain a solution. To the obtained solution, 35 wt% hydrochloric acid was added as a catalyst so that the hydrochloric acid concentration was 0.2 wt%. Then, the temperature was adjusted to 15°C, and 22 parts by weight of n-butyraldehyde was added while stirring. Next, 148 parts by weight of n-butyraldehyde was added to precipitate a white particulate polyvinyl butyral resin. Then 15 minutes after precipitation, 35 wt% hydrochloric acid was added so that the hydrochloric acid concentration became 1.8 wt%, then heated to 50°C, and held at 50°C for 2 hours. Subsequently, the solution was cooled and neutralized, and then the polyvinyl butyral resin was washed with water and dried to obtain a resin X1 that is a polyvinyl butyral resin.

The details of the obtained polyvinyl butyral resin (the resin X1) are shown in Table 1.

The content of the structural moiety in the obtained synthetic resin was measured by $^1$H-NMR (nuclear magnetic resonance spectrum) after dissolving the synthetic resin in DMSO-D6 (dimethyl sulfoxide).

Experimental Example 2;

A test was performed in the same manner as in Example 1 except that 30 parts by weight of N-vinylpyrrolidone was added to 100 parts by weight of polyvinyl acetal (a resin X2). Details of the resin X2 are shown in Table 1.

Experimental Example 3;

A test was performed in the same manner as in Example 1 except that polyvinyl alcohol having an average polymerization degree of 250 and a saponification degree of 99 mol%, and containing 2 mol% of a structural unit having an amino group represented by Formula (3) below was used (a resin X3). Details of the resin X3 are shown in Table 1.

[Chemical 1]

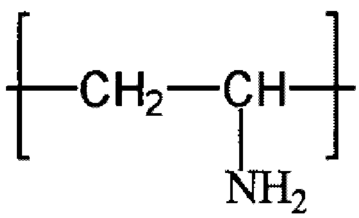

... (3)

Experimental Example 4;

To start with, 80 parts by weight of butyl methacrylate and 20 parts by weight of methoxyethyl acrylate were mixed to obtain a (meth)acrylic monomer solution. Next, 0.5 parts by weight of Irgacure 184 (manufactured by BASF) was dissolved in the obtained (meth)acrylic monomer solution, and the solution was applied onto a PET film. The applied product was irradiated with light having a wavelength of 365 nm at an integrated light amount of 2000 mJ/$cm^2$ using a UV conveyor device "ECS301G1" manufactured by EYE GRAPHICS Co., Ltd. at 25°C to obtain a poly(meth)acrylic acid ester resin solution. The obtained poly(meth)acrylic acid ester resin solution was vacuum-dried at 80°C for 3 hours to obtain a resin X4 as a poly(meth)acrylic acid ester resin. Details of the resin X4 are shown in Table 1.

Experimental Example 5;

Eighty-two parts by weight of a polyvinyl acetal resin obtained in the same manner as in the resin X1 and 9.5 parts by weight of acrylic acid (the linker) were dissolved in 15 parts by weight of THF, and reacted under ultraviolet irradiation for 20 minutes in the presence of a photoradical polymerization initiator to graft copolymerize the polyvinyl acetal resin and acrylic acid, thereby forming a linker moiety. One part by weight of the polyvinyl acetal resin having a linker moiety formed was dissolved in 99 parts by weight of butanol. After casting 10 μL of the obtained solution on the surface of a 12-well cell culture plate (manufactured by Corning Incorporated, area of bottom surface: 385 $mm^2$), the solution was spread over the whole and heated at 60°C for 60 minutes to obtain a resin film with a smooth surface.

A linear peptide having an amino acid sequence of Gly-Arg-Gly-Asp-Ser (number of amino acid residues: 5, described as GRGDS in the table) was prepared. Nine point five parts by weight of this peptide and 25 parts by weight of 1-ethyl-3-(3-dimethylaminopropyl) carbodiimide hydrochloride (a condensing agent) were added to phosphate buffered saline containing neither calcium nor magnesium so that the final concentration of the peptide was 1 mM, thereby preparing a peptide-containing solution. The peptide-containing solution (1660 parts by weight) was added to the cast resin film (a polyvinyl acetal resin having a linker moiety formed) and reacted to dehydrate and condense the carboxyl group of the linker moiety and the amino group of Arg of the peptide. In this way, a peptide-containing polyvinyl acetal resin having a polyvinyl acetal resin moiety, a linker moiety, and a peptide moiety (a resin X5) was produced. Details of the resin X5 are shown in Table 1.

Production of resin film 10;

The obtained resin (1 g) was dissolved in 19 g of butanol to obtain a resin solution. The obtained resin solution (150 μE) was discharged onto a φ22 mm cover glass (dust of 22 circle No. 1 manufactured by Matsunami Glass Ind., Ltd. was removed with an air duster and used) and rotated at 2000 rpm for 20 seconds using a spin coater to obtain a smooth resin film 10 (area in plan view: 3.8 $cm^2$, thickness: 20 nm). The obtained resin film 10 was disposed on the cell binding unit 6 together with the cover glass. Furthermore, in Experimental Example 5, 1 g of the polyvinyl acetal resin having a linker moiety formed was dissolved in 19 g of butanol, a resin film was obtained by the same operation, and then a peptide-containing liquid was added to prepare a peptide-containing polyvinyl acetal resin. In addition, in the Reference Experimental Example, a protein film was prepared using collagen (product name: Vitrigel).

[Evaluation]

(Surface free energy)

The surface free energy of the resin film (and the protein film) was measured using a contact angle meter (DMo-701 manufactured by Kyowa Interface Science Co., Ltd.). Pure water (1 uL) was dropped onto the resin film, and a droplet image after 30 seconds was photographed to obtain a contact angle of pure water. In addition, 1 μL of diiodomethane was dropped onto the resin film, and a droplet image after 30 seconds was photographed to obtain a contact angle of diiodomethane. From the obtained contact angle, the dispersion component $\gamma^d$ and the dipole component $\gamma^p$ of the surface free energy were calculated using the theoretical formula of Kaelble-Uy.

(Evaluation of adhesiveness)

HepG2 cells of a human liver cancer cell line were mixed with a culture solution so as to be $1.8 \times 10^5$ cells/mL to prepare a HepG2 cell suspension. By injecting 1 mL of the HepG2 cell suspension into the cell binding unit 6, HepG2 cells were seeded.

Evaluation was performed according to the following evaluation criteria.

<Uniformity>

A Cells are uniformly adhered
B There is unevenness in cell adhesion

<Adhesion maintainability>

The following are the criteria for the cells in the cell binding unit 6 when 1 mL of the culture medium in the liquid

medium reservoir 5 were delivered by irradiating the light irradiation unit 22 with UV 1 hour after seeding.

AA ··· 80% or more adhered
A ······ 50% or more and less than 80% adhered
B ······ 30% or more and less than 50% adhered

(Evaluation of handleability)

<Storage at normal temperature>

The storage of the microfluidic chip 1 was evaluated according to the following criteria.

A Storage at normal temperature possible
B Cold storage required

(Albumin secretion activity)

In order to evaluate the albumin secretion activity of the HepG2 cells cultured in the cell binding unit 6, the light irradiation unit 22 was irradiated with UV every 48 hours after culture, so that the medium in the liquid medium reservoir 5 was fed to the cell binding unit 6, and the medium in the cell binding unit 6 was fed to the recovery units 7a to 7c. The amount of albumin in the medium recovered in each of the recovery units 7a to 7c was detected by ELISA method.
The results are shown in Table 1 below.

[Table 1]

| | | Experimental Example 1 | Experimental Example 2 | Experimental Example 3 | Experimental Example 4 | Experimental Example 5 | Reference Experimental Example |
|---|---|---|---|---|---|---|---|
| | | Resin X1 | Resin X2 | Resin X3 | Resin X4 | Resin X5 | Collagen |
| Polyvinyl acetal skeleton | Acetalization degree (mol%) | 71 | 54 | 77 | - | 69 | - |
| | Acetyl group amount (mol%) | 1 | 1 | 1 | - | 3 | - |
| | Hydroxyl group amount (mol%) | 28 | 21 | 20 | - | 26 | - |
| | Total of structural units having amine, imine, or amide structure (mol%) | - | 24 | 2 | - | - | - |
| | Polymerization degree | 250 | 250 | 250 | - | 250 | - |
| Linker moiety | Acrylic acid (mol%) | - | - | - | - | 1 | - |
| Peptide moiety | GRGDS (mol%) | - | - | - | - | 1 | - |
| Poly(meth)acrylic acid ester skeleton | Butyl methacrylate unit (mol%) | - | - | - | 90 | - | - |
| | Methoxyethyl acrylate unit (mol%) | - | - | - | 10 | - | - |
| | Polymerization degree | - | - | - | 250 | - | - |
| Surface free energy | Dispersion component $\gamma^d$ | 32.5 | 36.2 | 34.2 | 38.9 | - | - |
| | Dipole component $\gamma p$ | 3.5 | 2.2 | 3.3 | 17.9 | - | - |
| Surface state of cell binding unit | - | Resin film | Resin film | Resin film | Resin film | Resin film | Protein film |
| Evaluation of adhesiveness | Uniformity | A | A | A | A | A | B |
| | Adhesion maintainability | A | A | A | B | AA | AA |
| Evaluation of handleability | Storage at normal temperature | A | A | A | A | A | B |

(continued)

| | | Experimental Example 1 | Experimental Example 2 | Experimental Example 3 | Experimental Example 4 | Experimental Example 5 | Reference Experimental Example |
|---|---|---|---|---|---|---|---|
| Albumin secretion activity ($\mu g/10^6$ cells/day) | Day 2 | 60 | 58 | 62 | 52 | 67 | 64 |
| | Day 4 | 53 | 56 | 60 | 47 | 72 | 68 |
| | Day 6 | 42 | 40 | 58 | 40 | 64 | 45 |

## EXPLANATION OF SYMBOLS

1: Microfluidic chip
1a: First main surface
1b: Second main surface
1c: First side surface
1d: Second side surface
1e: Third side surface
1f: Fourth side surface
2: Chip main body
3a: Substrate
3b: Cover member
4: Microchannel
5: Liquid medium reservoir
6: Cell binding unit
7a to 7d: Recovery unit
8: Optical gas generation tape
9a to 9d: Valve
10: Resin film
21: Cell analysis device
22: Light irradiation unit
23: Analysis unit

## Claims

**1.** A microfluidic chip comprising:

a chip main body; and
an optical gas generation tape attached to the chip main body and configured to generate a gas by light irradiation,
the chip main body including a microchannel, a liquid medium reservoir provided in the middle of the microchannel, and a cell binding unit provided on a downstream side of the liquid medium reservoir in the microchannel, and
a liquid medium stored in the liquid medium reservoir being supplied to the cell binding unit by the gas generated from the optical gas generation tape.

**2.** The microfluidic chip according to claim 1, wherein

the cell binding unit has a resin film,
a resin constituting the resin film contains a poly(meth)acrylic acid ester skeleton or a polyvinyl acetal skeleton,
a dispersion component $\gamma^d$ of surface free energy of the resin film is 24.5 mJ/m$^2$ or more and 45.0 mJ/m$^2$ or less, and
a dipole component $\gamma^p$ of the surface free energy of the resin film is 1.0 mJ/m$^2$ or more and 20.0 mJ/m$^2$ or less.

**3.** The microfluidic chip according to claim 1, wherein

the cell binding unit has a resin film, and
a resin constituting the resin film contains a poly(meth)acrylic acid ester skeleton or a polyvinyl acetal skeleton and a peptide moiety.

**4.** A cell analysis device comprising:

the microfluidic chip according to any one of claims 1 to 3;
a light irradiation unit configured to irradiate the optical gas generation tape with light; and
an analysis unit configured to analyze a cell culture solution that has passed through the cell binding unit.

**5.** A cell analysis system comprising:

the microfluidic chip according to any one of claims 1 to 3;
a light irradiation unit; and
an analysis unit,
wherein
the light irradiation unit irradiates the optical gas generation tape with light,
the liquid medium stored in the liquid medium reservoir is supplied to the cell binding unit by the gas generated from the optical gas generation tape by the light irradiation, and
the analysis unit analyzes the cell culture solution that has passed through the cell binding unit.

**6.** A cell analysis method that uses the microfluidic chip according to any one of claims 1 to 3, the method comprising the steps of:

binding a cell to the cell binding unit;
irradiating the optical gas generation tape with light;
supplying the liquid medium stored in the liquid medium reservoir to the cell binding unit; and
analyzing the cell culture solution that has passed through the cell binding unit.

[FIG. 1.]

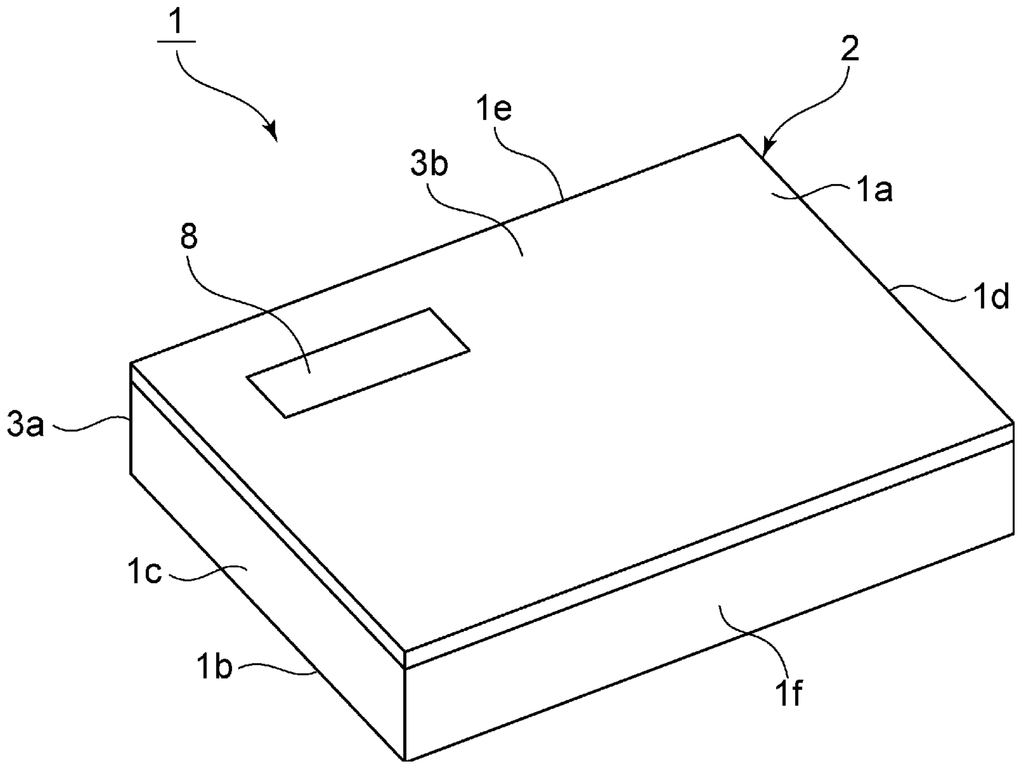
1
2
1e
3b
1a
8
1d
3a
1c
1b
1f

[FIG. 2.]

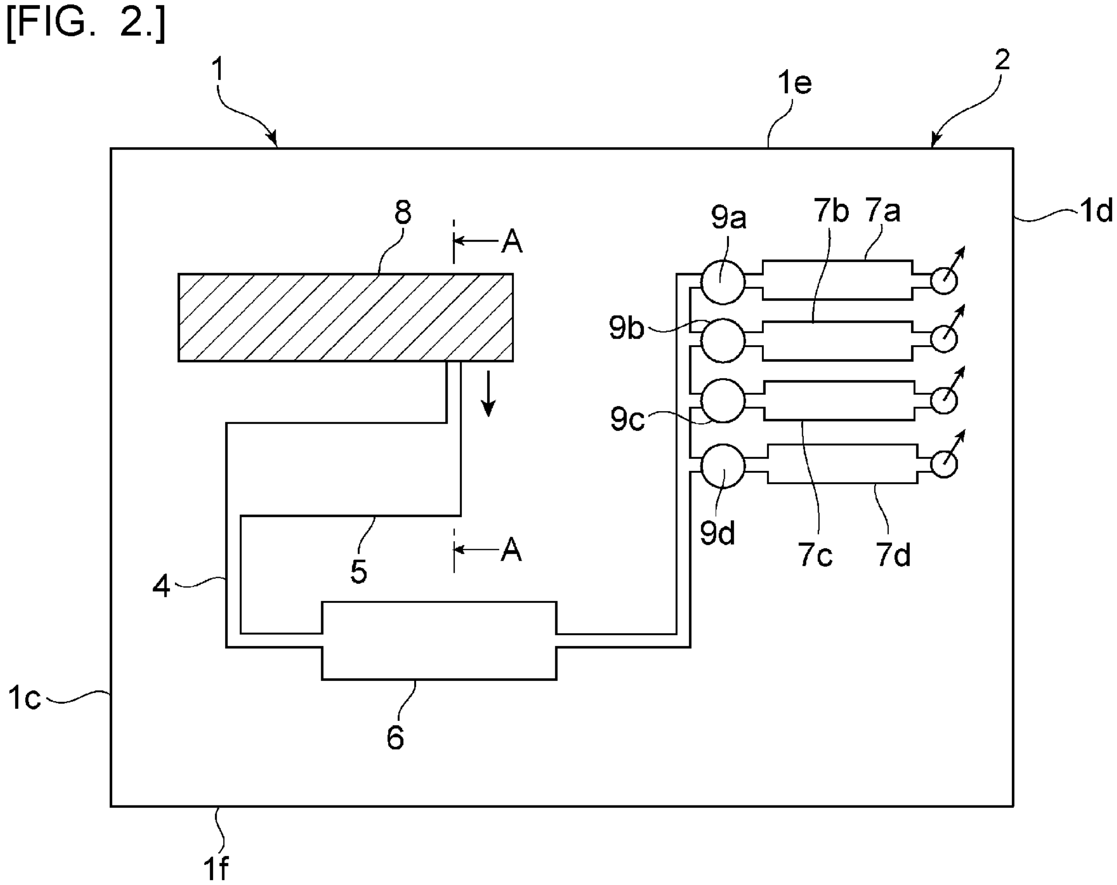
1
1e
2
8
A
9a
7b
7a
1d
9b
9c
9d
7c
7d
5
A
4
1c
6
1f

[FIG. 3.]

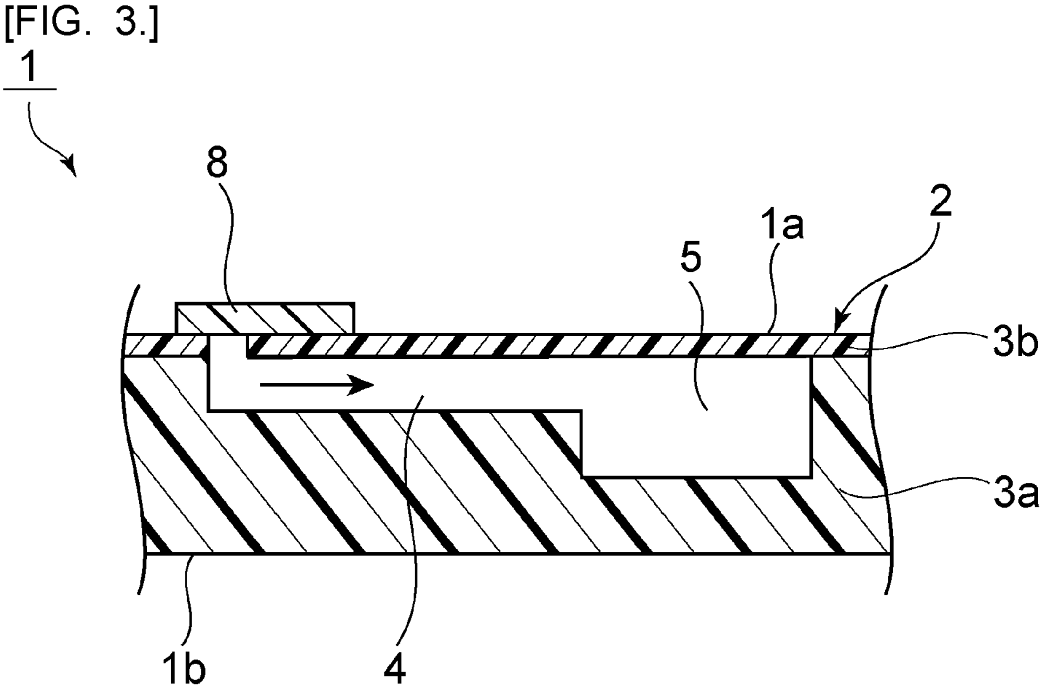


[FIG. 4.]

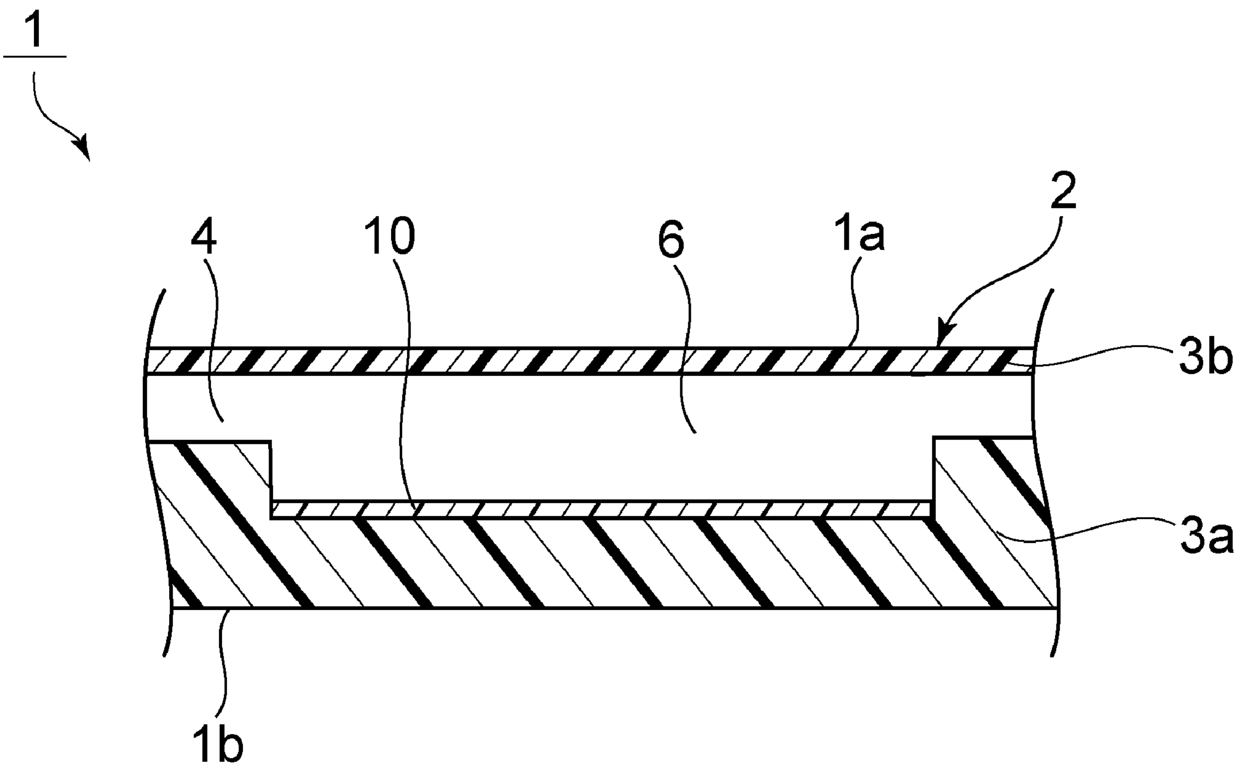

[FIG. 5.]

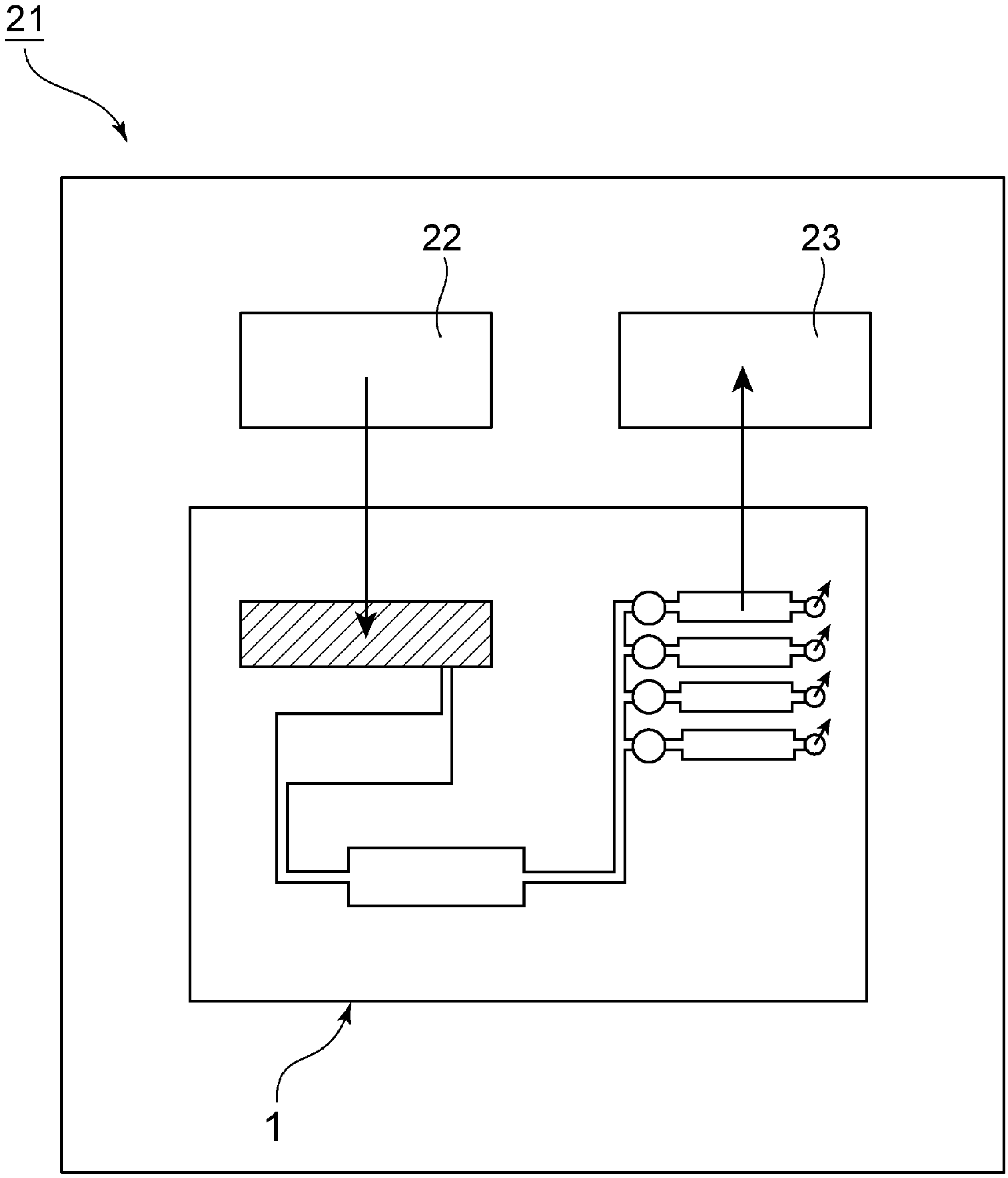

## INTERNATIONAL SEARCH REPORT

International application No. **PCT/JP2021/039652**

**A. CLASSIFICATION OF SUBJECT MATTER**

***C12M 1/00***(2006.01)i; ***C12Q 1/02***(2006.01)i
FI: C12M1/00 A ZNA; C12Q1/02

According to International Patent Classification (IPC) or to both national classification and IPC

**B. FIELDS SEARCHED**

Minimum documentation searched (classification system followed by classification symbols)

C12M1/00; C12Q1/02

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2018-513684 A (BERKELEY LIGHTS, INC) 31 May 2018 (2018-05-31) claims, fig. 1, 6 | 1-6 |
| Y | GOTO, Makiko et al. Development of a Microchip-Based Bioassay System Using Cultured Cells. Anal. Chem. 2005, vol. 77, pp. 2125-2131 abstract, fig. 1, 6 | 1-6 |
| Y | JP 2010-89259 A (SEKISUI CHEMICAL CO LTD) 22 April 2010 (2010-04-22) Background art, paragraph [0011], claims | 1-6 |
| Y | WO 2020/203769 A1 (SEKISUI CHEMICAL CO LTD) 08 October 2020 (2020-10-08) claims, paragraph [0011] | 2-6 |
| A | claims, paragraph [0011] | 1 |
| Y | JP 2009-538130 A (AGENCY FOR SCIENCE, TEHNOLOGY AND RESEARCH) 05 November 2009 (2009-11-05) abstract, claims | 2-6 |
| A | abstract, claims | 1 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 December 2021** | **28 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**

International application No.
**PCT/JP2021/039652**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

**INTERNATIONAL SEARCH REPORT**
**Information on patent family members**

International application No.
**PCT/JP2021/039652**

| Patent document cited in search report | Publication date (day/month/year) | Patent family member(s) | Publication date (day/month/year) |
|---|---|---|---|
| JP 2018-513684 A | 31 May 2018 | US 2016/0312165 A1<br>claims, fig. 1, 6<br>WO 2016/172454 A1<br>EP 3286296 A1<br>KR 10-2017-0140305 A<br>CN 107849505 A | |
| JP 2010-89259 A | 22 April 2010 | US 2011/0014096 A1<br>Background art, paragraph [0015], claims<br>EP 2256349 A1<br>KR 10-2010-0137410 A<br>CN 101978173 A | |
| WO 2020/203769 A1 | 08 October 2020 | (Family: none) | |
| JP 2009-538130 A | 05 November 2009 | US 2011/0053783 A1<br>abstract, claims<br>WO 2007/136354 A1<br>EP 2027257 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016104541 A


### Non-patent literature cited in the description


- *The Journal of Cell Biology,* September 1995, vol. 130 (5), 1189-1196
- *Protein, Nucleic Acid and Enzyme,* 2000, vol. 45 (15), 2477
- *Pathophysiology,* 1990, vol. 9 (7), 527-535
- *Osaka Women's and Children's Hospital Journal,* 1992, vol. 8 (1), 58-66